# EUROPÄISCHE PATENTSCHRIFT

(11) **EP 0 704 281 B1**
(45) Veröffentlichungstag und Bekanntmachung des Hinweises auf die Patenterteilung: **19.08.1998**
(21) Anmeldenummer: 95810559.5
(22) Anmeldetag: 08.09.1995
(51) Int. Cl.: B25B 23/142, A61C 8/00

(54) **Chirurgischer Drehmomentschlüssel mit einem Drehmomentindikator**
Surgical torque wrench with torque indicator
Clé dynamométrique chirurgicale avec indicateur de couple

(30) Priorität: 27.09.1994 CH 2921/94
(43) Veröffentlichungstag der Anmeldung: 03.04.1996
(73) Patentinhaber: INSTITUT STRAUMANN AG, 4437 Waldenburg (CH)
(72) Erfinder: Vogt, Martin, CH-4437 Waldenburg (CH); Schmutz, Werner, CH-4435 Niederdorf (CH); Schürch, Hans, CH-4425 Titterten (CH); Stadelmann, Eduardo, CH-1004 Lausanne (CH)
(74) Vertreter: Ullrich, Gerhard, Dr.

(56) Entgegenhaltungen:
- EP-A- 0 393 852
- DE-A- 3 026 134
- DE-A- 4 200 364
- FR-A- 1 498 385
- US-A- 2 447 109
- US-A- 2 691 295
- US-A- 2 936 661
- US-A- 3 587 307
- US-A- 5 345 845

## Beschreibung

### Anwendungsgebiet der Erfindung

Die Erfindung betrifft einen in der Chirurgie einsetzbaren Drehmomentschlüssel, der aus einem Drehmomentinstrument und einem daran als Zubehör ansetzbaren Drehmomentindikator besteht.

Beispielsweise ist es aus der Zahnmedizin bekannt, Implantate (Primärteile) in den Kieferknochen einzusetzen und auf den Implantaten durch Verschrauben Verbindungselemente, nämlich spezielle Schrauben und Suprastrukturen zu befestigen. Langzeitstabilität und Zuverlässigkeit der Verschraubungen hängen wesentlich davon ab, dass die Verschraubungen mit dem jeweils optimalen Drehmoment ausgeführt wurden. Ein zu schwaches Anziehen, d.h. zu geringes Drehmoment, kann später zur Lockerung der Verschraubungen führen. Ein zu starkes Anziehen, d.h. ein zu grosses Drehmoment, überlastet die eingesetzten Verbindungselemente, erhöht deren Bruchgefahr bzw. gefährdet die sichere Knochenverankerung des Implantats.

### Stand der Technik

Chirurgische Drehmomentschlüssel, z.B. in Gestalt einer Ratsche, weisen in herkömmlicher Ausbildung keinen zusätzlichen Drehmomentindikator bzw. keine Drehmoment-Begrenzungsmechanik auf (vgl. Spiekermann, H.: Farbatlanten der Zahnmedizin, Bd. 10 Implantologie. Georg Thieme Verlag, Stuttgart 1994, S.53). Ferner gibt es Schlüssel, die mit einer mechanischen oder elektronischen Drehmoment-Begrenzung ausgestattet sind. Bei mechanischen Drehmomentinstrumenten ist zumeist ein Mechanismus vorgesehen, der einen schwenkbaren Hebel oder eine drehbare Klinke am Ratschenkopf aufweist, so dass beim Überschreiten des zulässigen Drehmoments die Kraft einer vorgespannten Druckfeder überwunden wird und somit der Hebel durchknickt bzw. die Klinke ausrastet. Ein Drehmomentschlüssel mit einer Rutschkupplung ist bei Spiekermann a.a.O., Seite 33, offenbart.

Die soweit bekannten Drehmomentinstrumente besitzen den Nachteil, dass mit einer bestimmten Druckfeder nur ein relativ kleiner Bereich des Drehmoments M determiniert ist, z.B. mit M ≤ 18Ncm. In der Implantologie sind jedoch teils Drehmomente M = 30Ncm; bei bestimmten Implantatsystemen sogar Drehmomente M = 45Ncm und M = 60Ncm erforderlich. Verzichtet man auf die Einstellbarkeit des Drehmoments, so muss für jedes begrenzte Drehmoment ein fix eingestelltes Instrument zur Verfügung stehen. Dies bedeutet für den Anwender die Notwendigkeit, ein grösseres Sortiment von Instrumenten anzuschaffen, wodurch erhöhte Ausgaben verursacht werden. Zudem ist die Verwendung einer Druckfeder zur Drehmoment-Begrenzung ohnehin nicht ideal. Der Einsatz einer Druckfeder bedeutet Formschluss zwischen einem beweglichen Teil (z.B. einem Schwenkarm) und einem festen Teil (z.B. einer Schnappkugel), wobei Reibungskräfte zu überwinden sind, da der schwenkbare Arm über die die Druckkraft übertragende Kugel gleiten muss. Die Reibungsverhältnisse verändern sich jedoch rasch in unbestimmter Weise als Folge von aufgerauhten Oberflächen, Ablagerungen etc. Die bekannten Drehmomentinstrumente mit einer Drehmoment-Begrenzung sind sehr empfindlich und besitzen zum Teil Partien, welche sich nur mit übermässigem Aufwand reinigen lassen.

Aufgrund der vorgenannten störenden Einflüsse werden die Genauigkeit sowie die Reproduzierbarkeit der Drehmoment-Begrenzung negativ beeinflusst. Tests mit herkömmlichen Drehmomentinstrumenten ergaben Ungenauigkeiten von ± 10% im Bereich 20Ncm ≤ M ≤ 60Ncm. Zwar wird bei vielen Drehmomentinstrumenten das Überschreiten des an sich vorgesehenen Drehmoments erkennbar gemacht, z.B. durch Ausrasten, Einknicken oder ein Geräusch, die exakte Einhaltung der Drehmoment-Begrenzung ist jedoch nicht gewährleistet. Ausserdem ermöglichen diese Instrumente nicht, beim Anziehen, z.B. einer Schraube, den Anstieg des Drehmoments abzulesen, d.h. mitzuverfolgen.

An elektrischen Drehmomentinstrumenten ist es zumeist möglich, verschiedene Werte der Drehmoment-Begrenzung einzustellen und den Momentanwert des Drehmoments abzulesen. Derartige elektrische Instrumente sind aber um ein Vielfaches teurer als mechanische. Zudem können vielfach auch an elektrischen Instrumenten grössere Ungenauigkeiten auftreten. So wurde bei einem Instrument mit Rutschkupplung ein Fehler von ± 10% im Bereich 0Ncm ≤ M ≤ 45Ncm ermittelt. Da diese Toleranzen für den Anwender kaum überprüfbar sind, können Verschraubungen mit den Instrumenten nicht optimal ausgeführt werden. So wurden bei Nachuntersuchungen von Patienten mit implantatgestütztem Zahnersatz gelockerte Verschraubungen festgestellt, wodurch die gesamte Rekonstruktion gefährdet sein kann.

Mit chirurgischen Drehmomentinstrumenten muss folglich ein präzises, kontrolliertes Arbeiten möglich sein. Sie müssen so beschaffen sein, dass beim Eindrehen eines Verbindungselements, z.B. einer Schraube, diesem eine definierte Spannung verliehen wird. Ist eine komplexere Prothese an einem Patienten mit mehreren Verschraubungen vorzusehen, so werden wegen der gleichmässigen Spannungsverteilung alle Verschraubungen mit dem gleichen Anzugsdrehmoment auszuführen sein. Beispielsweise dürfen sich die Schraubverbindungen zwischen einem Dentalimplantat und einer Suprakonstruktion weder selbst lösen, noch darf es durch Überdrehen zu einem Bruch von Schrauben, anderen Konstruktionselementen oder zu Schäden am Knochen kommen.

Vom Maschinenbau her sind Drehmomentschlüssel mit einem Drehmomentindikator bekannt. In der FR-A-1 498 385 ist ein Drehmomentschlüssel mit einem Basisteil beschrieben, an dem sich ein konturiertes Mitnehmerelement befindet, wobei sich vom Basisteil zwei zueinander parallel verlaufende Biegestäbe mit unterschiedlichem Elastizitätsmodul erstrecken. Die Auslenkung des jeweils betätigten Biegestabes ist ein Mass für das ausgeübte Drehmoment, welches an einer Skala ablesbar ist. Ein erster Biegestab ist fest mit dem Skalenträger verbunden, während der zweite Biegestab die Nut des Skalenträgers durchragt und darin längsverschiebbar geführt wird. Beiderseits der Nut ist je eine dem betreffenden Biegestab zugeordnete Skala aufgetragen. Erfolgt der Kraftansatz am ersten Biegestab, so wird dieser gekrümmt und mit diesem der Skalenträger weggezogen, wodurch der zweite, unbetätigte Biegestab in die Nut einfährt und dabei die Grösse des Drehmoments anzeigt. Wirkt die Kraft auf den zweiten Biegestab ein, so wird dieser in Richtung der Nut gekrümmt.

Gemäss der US-A-3 587 307 ist an einem Drehmomentindikator eine Spanneinrichtung zur Fixierung eines Ringschlüssels vorgesehen. Von der Spanneinrichtung erstreckt sich ein Biegestab, an dessen anderen Ende fest ein vertikal ausgerichteter Skalenträger und abschliessend ein Handgriff vorgesehen sind. Von der Spanneinrichtung verläuft parallel zum Biegestab ein Zeiger hin zum Skalenträger. Der Handgriff und die Aufnahmeöffnung des Ringschlüssels liegen diametral gegenüber. Beim Ansetzen des Ringschlüssels an eine Schraube und Krafteinwirkung auf den Handgriff wird der Biegestab gekrümmt. Der Skalenträger wird ebenfalls ausgelenkt, während der Zeiger seine Position beibehält, so dass die Auslenkung aus der Nullposition als Mass für das ausgeübte Drehmoment ablesbar ist.

Die zuvor erwähnten, aus dem Maschinenbau stammenden Drehmomentschlüssel sind nicht durch blosses Miniaturisieren als Instrumente in der Chirurgie einsetzbar.

### Aufgabe der Erfindung

Angesichts der kritisch zu beurteilenden konstruktiven und funktionellen Merkmale, welche die existierenden chirurgischen Drehmomentschlüssel kennzeichnen, liegt der Erfindung das folgende Problem zugrunde. Zu schaffen ist ein Drehmomentschlüssel mit einem Drehmomentindikator, wobei sich der Gesamtaufbau aus weitgehend demontierbaren und möglichst einfachen Teilen zusammensetzen soll. Die individuelle Handhabung, fortschreitende Oberflächenrauhigkeit, Ablagerungen oder Korrosion dürfen sich nicht fehlerhaft auswirken. Gewünscht wird ein Drehmomentindikator, der mit existierenden Ratscheninstrumenten koppelbar ist. Hierbei müssen die ausgeübten Drehmomente kontrollierbar und reproduzierbar sein. Auf einer Skala, z.B. im Bereich 0Ncm ≤ M ≤ 40Ncm, möchte man das sich ändernde Drehmoment stets wertmässig ablesen können. Für das maximal ausgeübte Anzugsmoment liegt die Verantwortung beim Anwender, so dass dieser die Richtwerte des Lieferanten der Verbindungselemente und seine eigenen Erfahrungswerte nach der individuellen Situation berücksichtigen kann. Schliesslich müssen es die geometrischen Abmessungen und die Form des kompletten Drehmomentschlüssels erlauben, das Instrument an den verschiedenen Positionen im Mund des Patienten problemlos zu handhaben.

### Die Erfindung

Die vorgenannte Aufgabe wird durch den erfindungsgemässen Drehmomentschlüssel mit Drehmomentindikator gelöst, wie er im unabhängigen Patentanspruch 1 definiert ist. Bevorzugte Ausführungsvarianten ergeben sich aus den abhängigen Patentansprüchen 2 bis 5.

### Zeichnungen und Ausführungsbeispiel

Anhand der beiliegenden Zeichnungen erfolgt nachstehend die detaillierte Beschreibung eines Ausführungsbeispiels des erfindungsgemässen Drehmomentschlüssels mit Drehmomentindikator, wobei abschliessend mögliche Modifikationen erwähnt werden. Es zeigen:
- Figur 1a: in Frontalansicht einen Teilschnitt mit einem Drehmomentschlüssel in Form eines Ratscheninstruments, darin steckendem Eindrehwerkzeug und an dieses zuoberst angesetztem Führungsschlüssel sowie dem auf das Ratscheninstrument aufgestecktem Drehmomentindikator;
- Figur 1b: die Draufsicht gemäss Figur 1a;
- Figur 1c: als Detail den Eingriff zwischen der Rastnase des Ratscheninstruments und einer Nut am Eindrehwerkzeug;

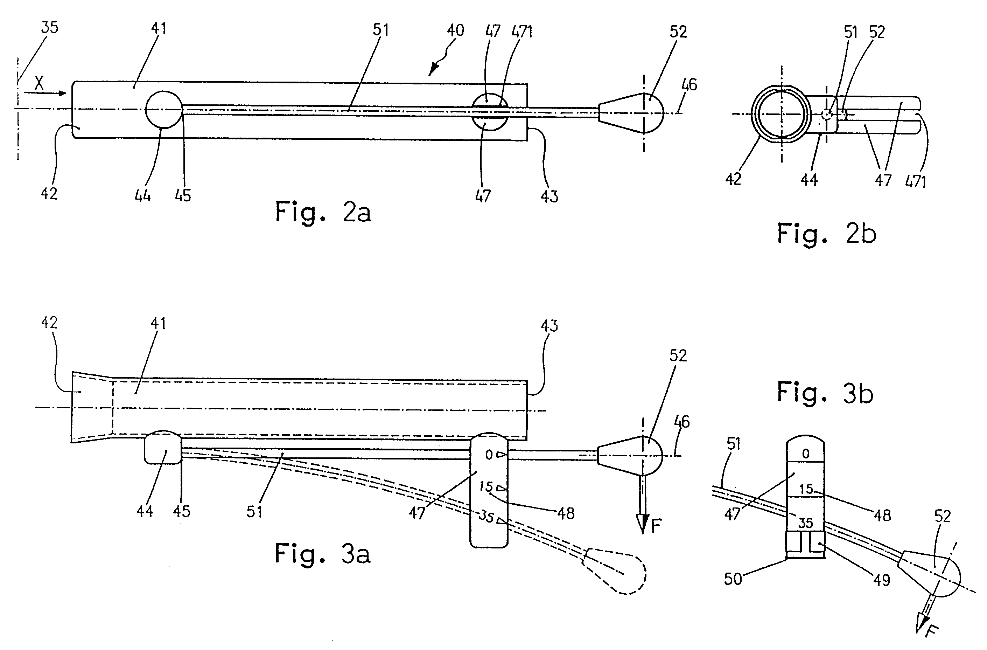

- Figur 2a: den separaten Drehmomentindikator in der Ansicht gemäss Figur 1a;
- Figur 2b: die Seitenansicht gemäss Figur 2a in der Pfeilrichtung X;
- Figur 3a: den separaten Drehmomentindikator in der Ansicht gemäss Figur 1b; und
- Figur 3b: die Skala des Drehmomentindikators mit einem Schleppzeiger.

### Figur 1a

Der komplett zusammengebaute Drehmomentschlüssel **1** besteht hier aus einem an sich bekannten Ratscheninstrument **10** und dem hülsenartig auf letzteres aufgeschobenen Drehmomentindikator **40**. Der Drehmomentindikator **40** wird im wesentlichen aus dem hülsenförmigen Träger **41** und dem Biegestab **51** gebildet. Im Ratschenkopf **11** des Ratscheninstruments **10**, nämlich in einer dafür vorgesehenen Vertikalbohrung **111**, steckt lösbar der Werkzeugkopf **31** des sich abwärts erstreckenden und an sich bekannten Eindrehwerkzeugs **30**, wobei der Werkzeugkopf **31** umfangsseitig mehrere vertikale Ratschennuten **32** aufweist. Der Ratschenkopf **11** ist zuvorderst an der zum Ratschengriff hinführenden Übergangspartie **121** angeordnet. Unterhalb des Werkzeugkopfes **31** schliesst sich ein Schaft **33** an, der mit der Schraubpartie **34** endet. Zuunterst ist in der Schraubpartie **34** eine Ausnehmung vorgesehen, in welcher der komplementäre Kopf der in das Implantat **3** einzudrehenden Schraube **2** aufgenommen wird. Ein Führungsschlüssel **4** ist an den Werkzeugkopf **31** angesetzt. Durch das Implantat **3**, die Schraube **2**, das Eindrehwerkzeug **30**, den Ratschenkopf **11** und die Kopfpartie des Führungsschlüssels **4** erstreckt sich die theoretische Drehachse **35**. Das Eindrehwerkzeug **30** ist auswechselbar; es wird je nach Kontur des Kopfes der Schraube **2** ausgewählt.

Für die gesamte weitere Beschreibung gilt folgende Festlegung. Sind in einer Figur zum Zweck zeichnerischer Eindeutigkeit Bezugsziffern enthalten, aber im unmittelbar zugehörigen Beschreibungstext nicht erläutert, so wird auf deren Erwähnung in vorangehenden Figurenbeschreibungen Bezug genommen.

### Figuren 1b und 1c

Das Ratscheninstrument **10** besteht prinzipiell aus zwei Baueinheiten, nämlich zum einen aus dem Ratschengriff **12** mit dem vorn an die Übergangspartie **121** angesetzten Ratschenkopf **11** und zum anderen aus der Bolzenstange **15** mit dem äusseren Drehknopf **25**, der Schraube **22** sowie der Druckfeder **21**. Jeweils auf der Ober- und Unterseite des Drehknopfes **25** ist ein in gleiche Richtung zeigender Richtungspfeil **26** aufgetragen. Durch den Ratschengriff **12** erstreckt sich eine axial durchgehende Bohrung **13**, die in der zur Aufnahme des Werkzeugkopfes **31** im Ratschenkopf **11** vorgesehenen Vertikalbohrung **111** mündet. Am freien Ende des Ratschengriffs **12**, welches dem Ratschenkopf **11** gegenüberliegt, tritt die Bohrung **13** aus, wobei der Austritt der Bohrung **13** als Innengewindeabschnitt **24**, komplementär zum Aussengewindeabschnitt **28** der Schraube **22**, ausgebildet ist. Im vorderen, dem Ratschenkopf **11** zugewandten Bereich besitzt der Ratschengriff **12** eine in die Bohrung **13** hineinragende Keilausformung **14**. Die Keilausformung **14** könnte durch äusserliches Einprägen auf den Ratschengriff **12** gebildet werden. Die Übergangspartie **121** zwischen dem Ratschenkopf **11** und dem Ratschengriff **12** hat eine nicht-rotationssymmetrische Kontur.

Die Bolzenstange **15** hat vorn, einstückig mit dem Schaft **27**, eine Instrumentenklinke **16**, an deren Stirnseite sich eine keilförmige Rastnase **161** befindet. Am Übergang zwischen der Instrumentenklinke **16** und dem verdickten Schaft **27** gibt es einen Absatz **162**. Der Schaft **27** besteht aus einem vorderen Abschnitt **271** und einem längeren hinteren Abschnitt **272**, zwischen denen eine radial umlaufende Nut **273** liegt. Am vorderen Abschnitt **271** sind zwei sich parallel gegenüberstehende, längsverlaufende und etwa bis auf den Durchmesser der Instrumentenklinke **16** reichende Abflachungen **19, 19'** vorgesehen. Diese Abflachungen **19, 19'** setzen sich hinter der Nut **273** fort und erstrecken sich partiell über den hinteren Abschnitt **272**. Der hintere Abschnitt **272** endet mit einer radial umlaufenden Schulter **20**, wodurch sich der Durchmesser der Bolzenstange **15** verringert, die sich von der Schulter **20** als Endabschnitt **29** fortsetzt. Auf dem Endabschnitt **29** sitzt die Druckfeder **21**, dahinter die drehbewegliche Schraube **22** und abschliessend der Drehknopf **25**. Die Druckfeder **21** stösst einerseits gegen die Schulter **20** und andererseits gegen den Aussengewindeabschnitt **28** der Schraube **22**. Zum Drehknopf **25** hin weist die Schraube **22** einen an den Aussengewindeabschnitt **28** ansetzenden, scheibenförmigen und den Ratschengriff **12** überragenden Schraubenkopf **23** auf.

Ist die Bolzenstange **15** in den Ratschengriff **12** eingesetzt, so greifen der Innengewindeabschnitt **24** des Ratschengriffs **12** und der Aussengewindeabschnitt **28** der Schraube **22** ineinander. Der mit Fingern fassbare, verdickte Schraubenkopf **23** liegt am Ende des Ratschengriffs **12** an und der Endabschnitt **29** durchragt den Aussengewindeabschnitt **28** sowie den Schraubenkopf **23**, so dass auch der Drehknopf **25** mit den Fingern ergriffen werden kann. Die Bolzenstange **15** steckt in der im Ratschengriff **12** vorgesehenen Bohrung **13** und die zuvorderst an der Instrumentenklinke **16** angeordnete Rastnase **161** ragt in die sich im Ratschenkopf **11** befindende Vertikalbohrung **111** hinein. Das Vordringen der Instrumentenklinke **16** in Richtung des Ratschenkopfes **11** wird dadurch begrenzt, dass der Absatz **162** im Inneren des Ratschengriffs **12** an eine Gegenschulter stösst. Ferner sitzt die Keilausformung **14** je nach Drehstellung der Bolzenstange **15** auf einer der Abflachungen **19, 19'** am hinteren Abschnitt **272** des Schafts **27**. Somit ist die Bolzenstange **15** drehgesichert und die Rastnase **161** verläuft parallel zur Drehachse **35**.

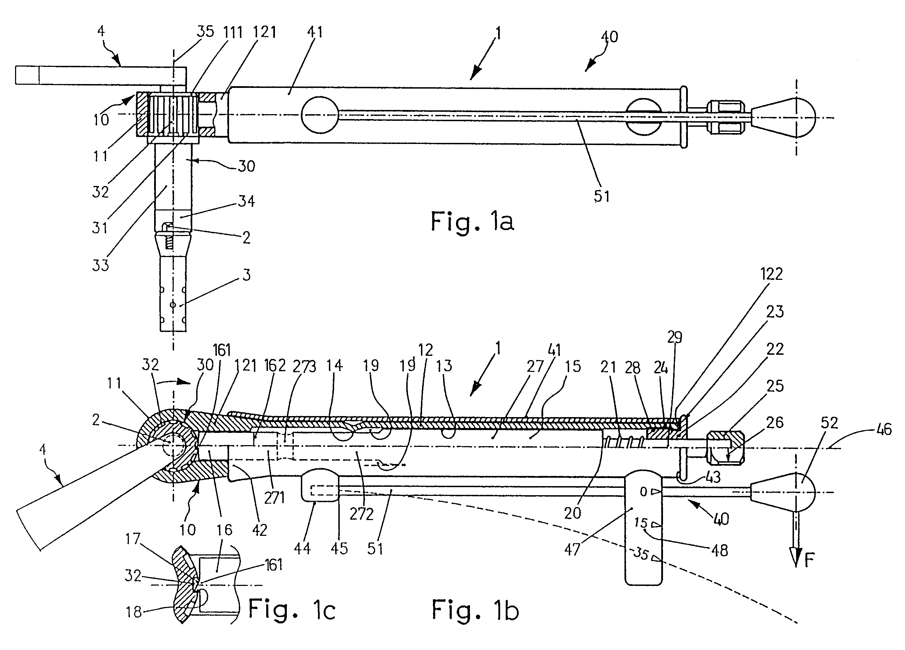

Steckt im Ratschenkopf **11** ein Eindrehwerkzeug **30**, so greift die über die Bolzenstange **15** unter Spannung der Druckfeder **21** stehende Rastnase **161** in eine am Werkzeugkopf **31** vorgesehene Ratschennut **32** ein, wodurch das Eindrehwerkzeug **30** in der Vertikalbohrung **111** gehaltert wird. Die Rastnase **161** ist asymmetrisch; sie besitzt im Verhältnis zur Drehachse **35** eine angeschrägte Ratschenflanke **17** sowie eine etwa rechtwinklig dazu ausgerichtete Blockierflanke **18**. Entsprechend dem Richtungspfeil **26** auf dem Drehknopf **25** ist die Bolzenstange **15** in zwei um 180° zueinander versetzte Drehstellungen im Ratschengriff **12** fixierbar. Folglich greift die Rastnase **161** in Richtung des Uhrzeigersinns oder gegenläufig in eine Ratschennut **32** am Werkzeugkopf **31** ein und in entsprechender Richtung sind die Ratschenflanke **17** bzw. die Blockierflanke **18** ausgerichtet. Der Werkzeugkopf **31** ist nicht entgegen der Blockierflanke **18** drehbar bzw. der Werkzeugkopf **31** wird bei einer Schwenkbewegung des Ratscheninstruments **10** in dem Drehsinn mitgenommen, nach welchem die Blockierflanke **18** hinweist. Schwenkt man das Ratscheninstrument **10** in der anderen Richtung, so gleitet die schräge Ratschenflanke **17** über die Ratschennuten **32** des feststehenden Eindrehwerkzeugs **30**. Bei jedem Überspringen der Rastnase **161** von einer Ratschennut **32** in die benachbarte wird die Bolzenstange **15** entgegen der Spannung der Druckfeder **21** minimal verschoben. In der beispielhaften Drehstellung gemäss Figur 1c - diese entspricht auch der Orientierung des Richtungspfeils **26** - greift die Rastnase **161** im Uhrzeigersinn in den Werkzeugkopf **31** ein, wodurch das Eindrehwerkzeug **30** bei einer Schwenkbewegung in Richtung des Uhrzeigersinns mitgenommen wird. Führt man das Ratscheninstrument **10** entgegen dem Uhrzeigersinn zurück, wird das Eindrehwerkzeug **30** nicht mitgenommen, die Rastnase **161** aber setzt in einer rückläufigen Ratschennut **32** erneut an.

Die Umkehrung der Mitnahmefunktion des Ratscheninstruments **10** ist folgendermassen einstellbar. Hierzu muss man die Bolzenstange **15** um 180° drehen, so dass der untere Richtungspfeil **26** nach oben kommt und aufwärts zeigt. Dazu ist die Bolzenstange **15** zunächst durch Erfassen des Drehknopfes **25** bis zum Anschlag aus dem Ratschengriff **12** herauszuziehen; erst dann lässt sich die Bolzenstange **15** um 180° drehen. Mit dem Herausziehen der Bolzenstange **15** gleitet die Abflachung **19** am hinteren Abschnitt **272** des Schafts **27** unter der Keilausformung **14** weg, bis die Keilausformung **14** in den Bereich der Nut **273** gelangt. In dieser Auszugsposition lässt sich die Bolzenstange **15** drehen. Massgeblich ist jedoch die Drehung um 180°, so dass jetzt die andere Abflachung **19'** der Keilausformung **14** zugewandt ist. Nun wird der Drehknopf **25** losgelassen, worauf die Druckfeder **21** die Bolzenstange **15** wieder in den Ratschengriff **12** hineinzieht und sich zugleich die Abflachung **19'** am hinteren Abschnitt **272** unter die Keilausformung **14** schiebt. Die Abflachungen **19**, **19'** am vorderen Abschnitt **271** haben nur die Funktion einer Führung und dass der vordere Abschnitt **271** beim Einschieben der Bolzenstange **15** die Keilausformung **14** passieren kann.

Mittels des Ratscheninstruments **10** ist ein Drehmoment auf das Eindrehwerkzeug **30** übertragbar, wobei die Funktion des Ratscheninstruments **10** reversibel ist, nämlich von einem Rechtsdrall-Drehmoment zu einem Linksdrall-Drehmoment, z.B. zum Anziehen einer Schraube **2** bzw. zum Lösen derselben.

Kombiniert mit dem Drehmomentindikator **40** ergibt das Ratscheninstrument **10** den erfindungsgemässen, betriebsbereiten Drehmomentschlüssel **1**. Der röhrchenförmige Träger **41** besitzt vorn eine erweiterte, zum partiellen Aufnehmen der Übergangspartie **121** komplementäre, d.h. formschlüssige Einführzone **42**. Ist der Träger **41** auf den Ratschengriff **12** aufgeschoben, so steckt der Ansatz der Übergangspartie **121** in der Einführzone **42**. Durch die nicht-rotationssymmetrischen Konturen der Übergangspartie **121** und der Einführzone **42** sind der Ratschengriff **12** und der Träger **41** gegeneinander drehgesichert. Der Trägerabschluss **43** erstreckt sich zumindest bis zum Griffabschluss **122**, wobei der Schraubenkopf **23** am Trägerabschluss **43** anschlägt. Bei angezogener Schraube **22** wird die Einführzone **42** auf den Ansatz der Übergangspartie **121** gedrückt.

Hinter der Einführzone **42** ist auf dem Träger **41** an der Einspannstelle **45** das fixe Ende des linear-elastischen Biegestabes **51** fest angeordnet. Vorzugsweise ist zum Fixieren des Biegestabes **51** ein am Träger **41** erhaben angeordneter Einspannkopf **44** vorgesehen. Der Biegestab **51** verläuft in der horizontalen Ebene parallel zur Längsachse **46**, erstreckt sich über den Drehknopf **25** hinaus und ist an seinem freien Ende mit einem Fingerglied **52** versehen. Nahe dem Trägerabschluss **43** ist senkrecht an den Träger **41** und senkrecht zum Biegestab **51** ausgerichtet, ein Steg **47** angesetzt, auf dem eine Masseinteilung **48** aufgetragen ist. Der Steg **47** hat zugleich eine Führungsfunktion für den beim Arbeiten ausgelenkten Biegestab **51**. Aus einer Nullstellung wird der Biegestab **51** mit einer am Fingerglied **52** angreifenden Kraft **F** nach vorn gezogen. Je nach Grösse der Kraft **F** verbiegt sich der Biegestab **51** bis zu dem adäquaten Wert auf der Masseinteilung **48**, die z.B. in Ncm geeicht ist. Der Biegestab **51** sollte keinen Kontakt mit dem Steg **47** haben, um Messwertfehler durch Reibung zu vermeiden.

Der Zusammenbau des Ratscheninstruments **10** und des Drehmomentindikators **40** geschieht folgendermassen: Das Ratscheninstrument **10** ist in den Ratschengriff **12** mit dem daran befindlichen Ratschenkopf **11** sowie die Bolzenstange **15** mit der daran angeordneten Druckfeder **21**, Schraube **22** und Drehknopf **25** zerlegt. Man schiebt den Träger **41** samt dem daran angebrachten Biegestab **51** mit der Einführzone **42** voran über den Ratschengriff **12** bis zum Formschluss zwischen der Einführzone **42** und der Übergangspartie **121**. Darauf wird die Bolzenstange **15** in die Bohrung **13** hineingeschoben, wobei eine der Abflachungen **19, 19'** unter die Keilausformung **14** gleitet. Schliesslich dreht man die Schraube **22** ein, so dass die Schraubverbindung zwischen dem Aussengewindeabschnitt **28** und dem Innengewindeabschnitt **24** hergestellt ist. Nach Überprüfen der gewünschten Drehstellung des Drehknopfes **25** ist der Drehmomentschlüssel **1** funktionsbereit.

### Figuren 2a bis 3a

Vorteilhafterweise sind zwei parallele, zueinander beabstandete Stege **47** vorgesehen, zwischen denen der Biegestab **51** hindurchragt, so dass der Biegestab **51** nur innerhalb des von den beiden Stegen **47** gebildeten Schlitzes **471** bewegt werden kann. Damit ist die Richtung für den Ansatz der Kraft **F** am Fingerglied **52** vorgegeben und ein verkanteter Gebrauch des Drehmomentschlüssels **1** ausgeschlossen.

Die Dimensionierung des Drehmomentschlüssels **1** lässt sich nach den bekannten Formeln vornehmen, wobei eine bestimmte Auslenkung des Biegestabes **51** einem analogen, auf das Eindrehwerkzeug **30** ausgeübten Drehmoment **M** entspricht.

### Figur 3b

In weiterer Ausgestaltung des Drehmomentindikators **40** sind an den Stegen **47** ein Schleppzeiger **49** zur Markierung der weitesten Auslenkung des Biegestabes **51** sowie ein Anschlag **50** zur Begrenzung seiner maximal möglichen Auslenkung vorgesehen. Für den Biegestab **51** hat sich besonders eine Legierung auf Co-Ni-Cr-Basis mit Be- und Ti-Zusätzen in Form eines glatten, im Kaltverfahren hartgezogenen, präzisionsgeschliffenen und vergüteten Drahtes von 1,25mm Durchmesser und mit einem E-Modul im Bereich von 215 bis 230kN/mm² bewährt.

Zu den vorangehenden Ausführungen sind weitere konstruktive Variationen realisierbar. Hier ausdrücklich erwähnt seien noch:
- Die Schraube **22** besitzt hinter dem bisherigen Schraubenkopf **23** noch ein Vielkantsegment, wenn das Anziehen der Schraube **22** mit einem Schraubenschlüssel gewünscht ist.
- Anstelle mit dem Ratscheninstrument **10** ist der Drehmomentindikator **40** auch mit einem äquivalenten Instrument kombinierbar, mit dem ein zu drehendes Werkzeug direkt oder indirekt geführt wird.

## Patentansprüche

1. Chirurgischer Drehmomentschlüssel (**1**) bestehend aus einem Drehmomentinstrument (**10**) und einem daran als Zubehör ansetzbaren Drehmomentindikator (**40**), **dadurch gekennzeichnet**, dass
a) der Drehmomentindikator (**40**) aus einem langgestreckten, steifen und am Drehmomentinstrument (**10**) zu montierenden Träger (**41**) gebildet wird und
b) längs des Trägers (**41**) ein linear-elastischer Biegestab (**51**) verläuft, der in einer am Biegestab (**51**) vorhandenen Einspannstelle (**45**) mit seinem einen Ende fest angeordnet ist und
c) das freie Ende des Biegestabes (**51**) zur Einwirkung einer vom Benutzer ausgeübten Kraft (**F**) vorgesehen ist und
d) sich direkt oder indirekt am Träger (**41**) eine Masseinteilung (**48**) befindet, an welcher die Auslenkung des Biegestabes (**51**) infolge der Krafteinwirkung als erzeugtes Drehmoment (**M**) ablesbar ist.

2. Drehmomentschlüssel (**1**) nach Anspruch 1, **dadurch gekennzeichnet**, dass
a) der Träger (**41**) als Hülse ausgebildet ist, welche mit der nicht-rotationssymmetrischen Einführzone (**42**) voran auf den Griff (**12**) des Drehmomentinstruments (**10**) axial aufschiebbar ist und
b) die Einführzone (**42**) die zwischen dem Griff (**12**) und dem Kopf (**11**) des Drehmomentinstruments (**10**) liegende Übergangspartie (**121**) formschlüssig aufnimmt und
c) der Träger (**41**) sich mit seinem Trägerabschluss (**43**) im Prinzip bis zum Griffabschluss (**122**) erstreckt und
d) der Träger (**41**) mit einer in den Griffabschluss (**122**) eindrehbaren Schraube (**22**) axial fixiert ist.

3. Drehmomentschlüssel (**1**) nach Anspruch 1, **dadurch gekennzeichnet**, dass
a) sich am Träger (**41**), nahe der Übergangspartie (**121**), ein Einspannkopf (**44**) befindet, in dem der Biegestab (**51**) fixiert ist und
b) am Träger (**41**), nahe dem Trägerabschluss (**43**), zumindest ein horizontal abstehender Steg (**47**) vorgesehen ist, welcher die Masseinteilung (**48**) aufweist und
c) sich der Biegestab (**51**) parallel zur durch den Drehmomentschlüssel (**1**) hindurchgehenden Längsachse (**46**) erstreckt und
d) der in der horizontalen Ebene - hierin liegt auch die Längsachse (**46**) - auslenkbare Biegestab (**51**) über den Steg (**47**) hinausragt.

4. Drehmomentschlüssel (**1**) nach Anspruch 3, **dadurch gekennzeichnet**, dass
a) am Träger (**41**) zwei zueinander parallele und horizontal abstehende Stege (**47**), die einen dazwischenliegenden Schlitz (**471**) bilden, vorgesehen sind, in welchem die hintere, freie Partie des Biegestabes (**51**) bewegbar ist und
b) an beiden Stegen (**47**) die Masseinteilung (**48**) aufgetragen ist und
c) wahlweise die maximale Auslenkung des Biegestabes (**51**) mittels eines an den Stegen (**47**) angeordneten Anschlages (**50**) begrenzt ist und/oder sich an den Stegen (**47**) ein Schleppzeiger (**49**) befindet und
d) auf das freie Ende des Biegestabes (**51**) ein Fingerglied (**52**) aufgesetzt ist.

5. Drehmomentschlüssel (**1**) nach Anspruch 1, **dadurch gekennzeichnet**, dass der Biegestab (**51**) aus einer Legierung auf Co-Ni-Cr-Basis mit Be- und Ti-Zusätzen in Form eines glatten, im Kaltverfahren hartgezogenen, präzisionsgeschliffenen und vergüteten Drahtes von 1,25mm Durchmesser und mit einem E-Modul im Bereich von 215 bis 230kN/mm² besteht.

## Claims

1. Surgical torque wrench (**1**) comprising a torque instrument (**10**) and a torque indicator (**40**), which is fixable to the torque wrench as an accessory, characterized in that
a) the torque indicator (**40**) is formed from an elongated, rigid support (**41**) to be mounted on the torque instrument (**10**), and
b) a linearly elastic bending rod (**51**) extends along the support (**41**), which is firmly arranged with one of its ends in a clamping site (**45**) present on the bending rod (**51**), and
c) the free end of the bending rod (**51**) is intended for the application of a force (**F**) exerted by the user, and
d) a graduation (**48**) is located directly or indirectly on the support (**41**) on which the deflection of the bending rod (**51**) as a consequence of the application of force can be read off as generated torque (**M**).

2. Torque wrench (**1**) according to Claim 1, characterized in that
a) the support (**41**) is designed as a sleeve which can be axially pushed, with the non-rotationally symmetrical insertion zone (**42**) at the front, onto the handle (**12**) of the torque instrument (**10**), and
b) the insertion zone (**42**) positively receives the transition region (**121**) lying between the handle (**12**) and head (**11**) of the torque instrument (**10**), and
c) the support (**41**) extends in principle by its support end (**43**) up to the handle end (**122**) and
d) the support (**41**) is axially fixed with a screw (**22**) that can be turned into the handle end (**122**).

3. Torque wrench (**1**) according to Claim 1, characterized in that
a) a clamping head (**44**) in which the bending rod (**51**) is fixed is located on the support (**41**) in the vicinity of the transition region (**121**), and
b) at least one horizontally projecting web (**47**) having the graduation (**48**) is provided on the support (**41**) in the vicinity of the support end (**43**), and
c) the bending rod (**51**) extends in parallel to the longitudinal axis (**46**) passing through the torque wrench (**1**), and
d) the bending rod (**51**) which can be deflected in the horizontal plane - which is the plane in which the longitudinal axis (**46**) also lies - projects beyond the web (**47**).

4. Torque wrench (**1**) according to Claim 3, characterized in that
a) two webs (**47**) which are parallel to one another and project horizontally and which form a slot (**471**) between them are provided on the support (**41**), in which slot the rear free part of the bending rod (**51**) can be moved, and
b) the graduation (**48**) is provided on both webs (**47**), and the maximum deflection of the bending rod (**51**) is selectively delimited by means of a stop (**50**) located on the webs (**47**) and/or a maximum indicator (**49**) is located on the webs (**47**), and
d) a finger member (**52**) is applied onto the free end of the bending rod (**51**).

5. Torque wrench (**1**) according to Claim 1, characterized in that the bending rod (**51**) is made of an alloy based on Co-Ni-Cr with additions of Be and Ti in the form of a smooth, precision-ground and quenched wire which is hard-drawn by a cold method and which has a diameter of 1.25 mm and a modulus of elasticity within the range from 215 to 230 kN/mm2.

## Revendications

1. Clé dynamométrique chirurgicale (1), constituée d'un instrument dynamométrique (10) et d'un indicateur de couple (40) pouvant être rattaché comme accessoire à cet instrument, **caractérisée** en ce que
a) l'indicateur de couple (40) est constitué d'un élément porteur (41) oblong et rigide, à monter sur l'instrument dynamométrique (10),
b) une barre de flexion (51) à élasticité linéaire s'étend le long de l'élément porteur (41), barre qui est disposée fixement par une extrémité, en un point de serrage (45) présent sur la barre de flexion (51), et
c) l'extrémité libre de la barre de flexion (51) est prévue pour l'action d'une force (F) exercée par l'utilisateur, et
d) une graduation (48) se trouve directement ou indirectement sur l'élément porteur (41), sur laquelle on peut lire, sous forme de couple produit (M), la déviation de la barre de flexion (51) sous l'action de la force.

2. Clé dynamométrique (1) selon la revendication 1, **caractérisée** en ce que
a) l'élément porteur (41) est réalisé sous forme de manchon, qui peut être enfilé axialement, avec sa zone d'introduction (42) sans symétrie de révolution en avant, sur la poignée (12) de l'instrument dynamométrique (10), et
b) la zone d'introduction (42) reçoit en engagement positif la partie de transition (121) située entre la poignée (12) et la tête (11) de l'instrument dynamométrique (10), et
c) l'élément porteur (41) s'étend en principe, par sa terminaison (43), jusqu'à la terminaison (122) de la poignée, et
d) l'élément porteur (41) est fixé en position axiale par une vis (22) qui peut être vissée dans la terminaison (122) de la poignée.

3. Clé dynamométrique (1) selon la revendication 1, **caractérisée** en ce que
a) une tête de serrage (44), dans laquelle la barre de flexion (51) est fixée en position, se trouve sur l'élément porteur (41) à proximité de la partie de transition (121), et
b) au moins une branche (47) horizontalement saillante, qui présente la graduation (48), est prévue sur l'élément porteur (41) à proximité de sa terminaison (43), et
c) la barre de flexion (51) s'étend parallèlement à l'axe longitudinal (46) traversant de part en part la clé dynamométrique (1), et
d) la barre de flexion (51), qui peut être déviée dans le plan horizontal - dans lequel se situe également l'axe longitudinal (46)-, s'étend au-delà de la branche (47).

4. Clé dynamométrique (1) selon la revendication 3, **caracterisée** en ce que
a) deux branches (47) horizontalement saillantes et parallèles sont prévues sur l'élément porteur (41), et elles forment entre elles une fente (471) dans laquelle est mobile la partie arrière libre de la barre de flexion (51), et
b) la graduation (48) est apposée sur les deux branches (47), et
c) la déviation maximale de la barre de flexion (51) est limitée au moyen d'une butée (50) disposée sur les branches (47) et/ou, au choix, une aiguille entraînée (49) se trouve sur les branches (47), et
d) un organe (52) de saisie par les doigts est monté à l'extrémité libre de la barre de flexion (51).

5. Clé dynamométrique (1) selon la revendication 1, **caractérisée** en ce que la barre de flexion (51) est constituée d'un alliage à base de Co-Ni-Cr avec des additifs de Be et de Ti, sous la forme d'un fil métallique lisse, étiré dur à froid, subissant une rectification de précision et trempé, de 1,25 mm de diamètre et avec un module d'élasticité dans la plage de 215 à 230 kN/mm².
